# EUROPEAN PATENT APPLICATION

(11) **EP 0 562 196 A1**
(43) Date of publication of application: **29.09.1993**
(21) Application number: 92311699.0
(22) Date of filing: 22.12.1992
(51) Int. Cl.: A63B 69/00

(54) **Advanced reaction training apparatus**

(30) Priority: 27.02.1992 US 843899
(71) Applicant: INNOVATIVE TRAINING PRODUCTS, INC., Syossett, New York 11791 (US)
(72) Inventor: Elstein, Rick A., Melville, New York 11747 (US); Kleger, Lawrence A., Floral Park, New York 11005 (US); Trager, Wesley, Bayville, New York 11709 (US)
(74) Representative: Read, Matthew Charles

(57) **Abstract**

An advanced reaction training apparatus for training, maintaining, developing or rehabilitating a user's reactionary movement skills having a plurality of stimulus indicators (22-24) for signaling designated reaction movement patterns, a microprocessor (26) with stored programs for controlling the operation of the apparatus and for providing stored function programs for user selection, and a memory for storing information including response-time data. Further processing of the response-time data into a reaction factor provides objective and qualitative feedback to the user.

## Description

### TECHNICAL FIELD

The present invention pertains in general to sports training systems and in particular relates to an Advanced Reaction Training apparatus for training, maintaining, developing or rehabilitating a user's reactionary movement skills.

### BACKGROUND OF THE INVENTION

Modem training or exercising devices have drawn upon sophisticated controls including stimulus and feedback indicators to allow a user to react and determine the timeliness of his reaction movement.

U.S. Patent 4,702,475 to Elstein et al. describes an apparatus for reaction training in which an array of lights is positioned visibly in front of a user. The lights are the reaction stimulus indicators with each light signifying a different movement pattern to be executed by the user within a measured time period. The apparatus signals the end of the time allotted to a particular movement by an audible beep.

U.S. Patent 4,834,375, also granted to Elstein et al., describes enhancement features of a reaction training apparatus including baseball batting tee(s), an optical sensor for detecting the hitting of a ball off of the tee and different audible signals to signal a hit or a miss at the end of a swing.

Although usage of these techniques and devices by users including professional athletes has yielded notably improved reactionary movement performance, an improved indication of the accuracy and quality of a user's response will better allow the user to objectively gauge his performance against others and to monitor the progress of his performance.

The present invention relates to an Advanced Reaction Trainer which in part includes many aspects and features described in U.S. patents 4,702,475 and 4,834,375. The disclosure of these patents are incorporated by reference herein.

### SUMMARY OF THE INVENTION

The Advanced Reaction Trainer ("ART") according to the present invention includes a plurality of lights for signalling different preassigned reactionary movement patterns; a keyboard for selecting functions and adjusting parameters; a display for displaying functions, parameters and data including response time in textual or numeric formats; a microprocessor for controlling the operations of the ART; and stored programs and data in memory for directing the operations of the ART, including recording a user's response time. The stored data may be downloaded to an external processing device such as a personal computer for further analyses or processing.

Another aspect of the present invention relates to an "acoustic stop" feature which includes an acoustic level detector for detecting the acoustic impact of the user's movement in response to a lamp signal. The acoustic detector can trigger a stop to a timer within one/thousandth of a second of the user's response. The use of the acoustic detector eliminates the need to rely on touch pads or light sensors, which may be cumbersome for a user.

Another technique of the present invention is a "count down" sequence, which simulates a live game situation such as a baseball pitcher's wind-up or a tennis opponent's swing prior to the contact of the ball. For example, a count down sequence of 3--2--1 with audible tones or with numeric display simulates the windup sequence and allows a user to "load up" his muscles to anticipate a movement signal. The use of the count down sequence in conjunction with the lamp signals is particularly beneficial because the user can simulate game situations where he anticipates when he has to react but does not know what response or direction will be called for. With the use of the count down sequence and the acoustic stop features, a user can drill test himself without an assistant. Baseball hitting and karate kicking are just two of the numerous moves that can be self-tested.

The Advanced Reaction Trainer according to the present invention also features an "assistant coach" device where the user performs movement sequences with another person acting as an assistant coach. The assistant coach device includes a directional stopwatch which is remotely connected to the ART and enables the assistant to select the lamp signals to start a movement and to stop a response-time timer when the movement is completed.

With the assistant coach, the user is not tied to any touch or sound sensors and therefore need not confine their movements within a specific location or reach a certain level of acoustic impact, enabling the assistant coach or the user to freely design movement patterns.

Still another feature of the present invention relates to the downloading of stored data including response-time data to an external device such as a personal computer for data processing, including quantification of the data into a reaction "factor". The factor provides the user with an objective criteria to compare his reaction performance against empirical data which correlates to game situations, or to compare against his own previously recorded personal data.

The empirical data factor correlates to game situations. For example, a reaction factor of 96 may indicate a player's ability to successfully react to the equivalent of pitches up to 96 miles per hour. A factor of 86 may indicate the ability to handle only pitches slower than 86 miles per hour.

Reaction factors can also be used to indicate the quality of a user's reactions, e.g. whether the reactions are within a desired range or reaction level. An example would be in reentry therapy when an injured athlete is put on a progressive schedule of performance by monitoring his improvements in reaction factors until he recovers his normal range or professional level. The use of reaction factors in the rehabilitation process helps to prevent re-injury from a user's unknowing over-stressing in training.

### BRIEF DESCRIPTION OF THE DRAWINGS

Fig. 1 is a block diagram of the Advanced Reaction Trainer according to the present invention.

Fig. 2 is a block diagram of a microprocessor according to an embodiment of the present invention.

Fig. 3 is a schematic diagram of an audio peak detector according to an embodiment of the present invention.

Fig. 4 is a flow diagram of the System Initialisation routine according to the present invention.

Fig. 5 is a flow diagram of the Select Lights routine according to the present invention.

Fig. 6 is a flow diagram of the Adjust Times initialization routine according to the present invention.

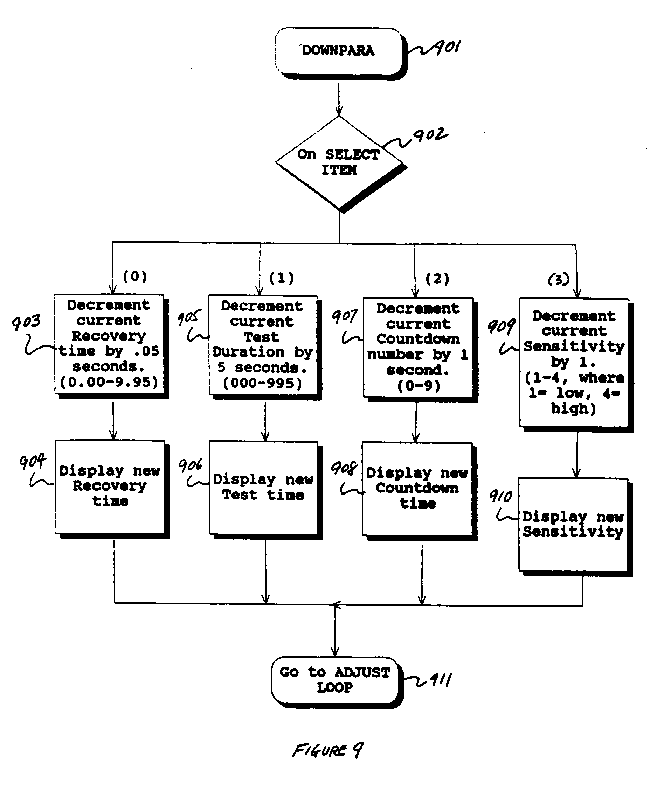

Fig. 7 is a flow diagram of the Parameter Adjust Initialization routine according to the present invention.

Fig. 8 is a flow diagram of the Increment Parameter routine according to the present invention.

Fig. 9 is a flow diagram of the Decrement Parameter routine according to the present invention.

Fig. 10 is a flow diagram of the Run Test Initialization routine according to the present invention.

Fig. 11 is a flow diagram of the Run Test routine according to the present invention.

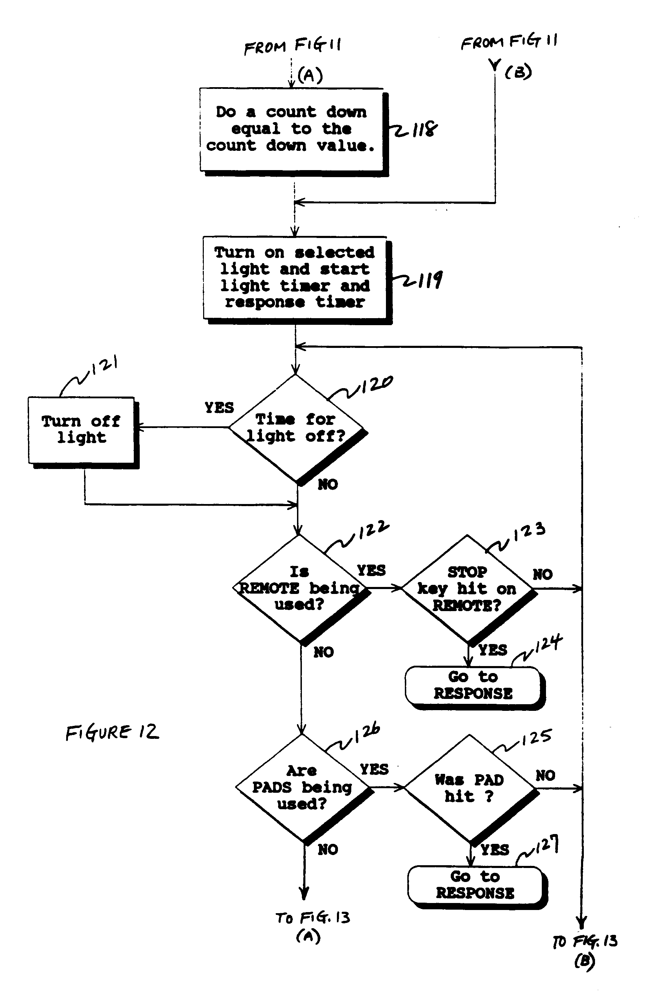

Fig. 12 is a continuing flow diagram of the Run Test routine according to the present invention.

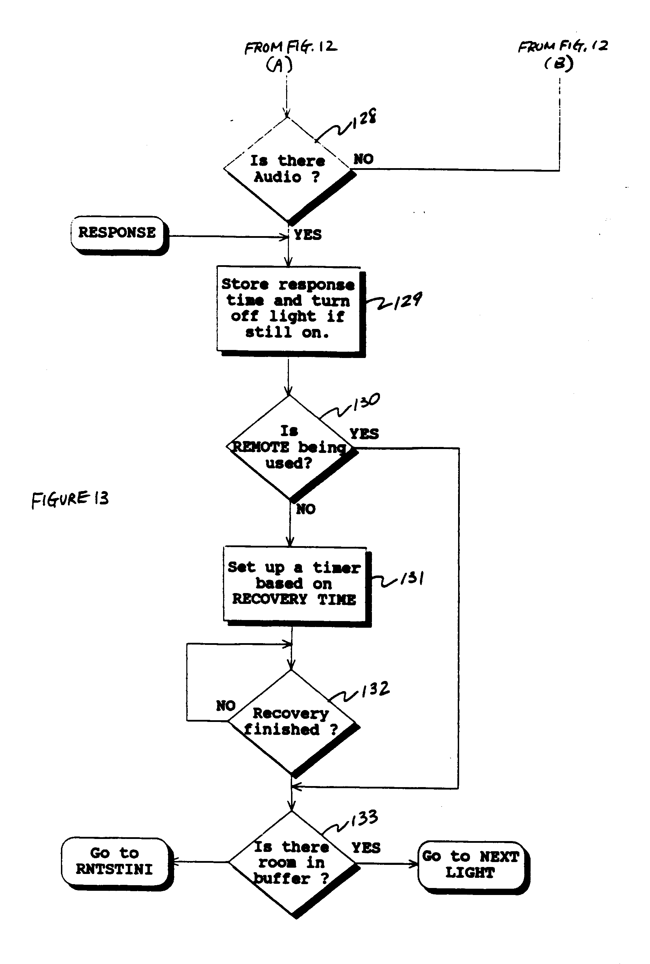

Fig. 13 is a continuing flow diagram of the Run Test routine according to the present invention.

Fig. 14 is a flow diagram of the Test Results Update routine according to the present invention.

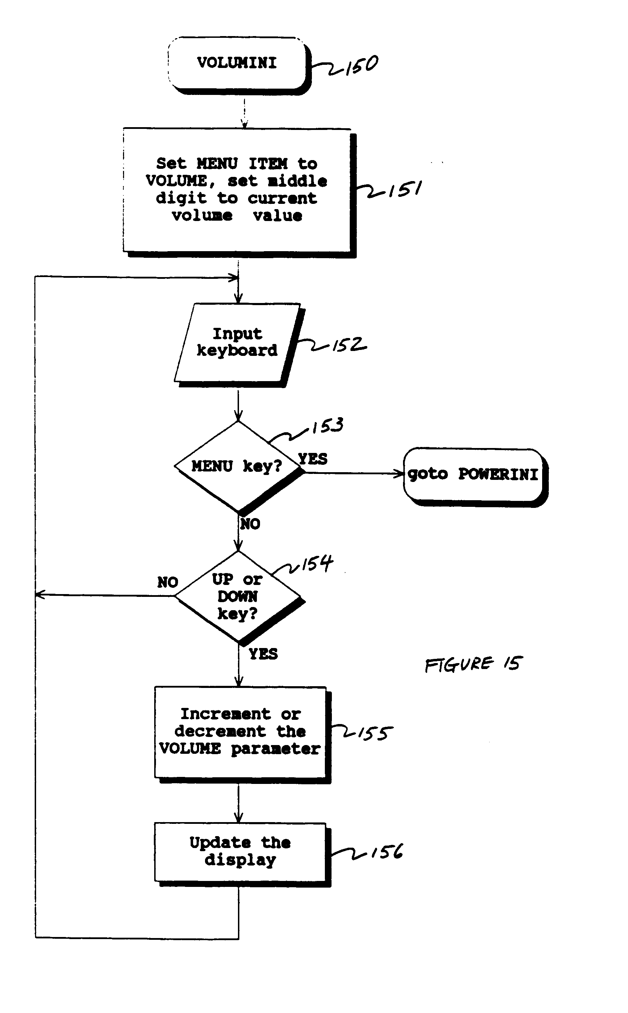

Fig. 15 is a flow diagram of the Volume Entry routine according to the present invention.

Fig. 16 is a flow diagram of the Power Initialiation routine according to the present invention.

Fig. 17 is a flow diagram of the Data Downloading routine according to the present invention.

Fig. 18 illustrates a format of data analysis including four test records.

Fig. 19 shows a converted data representing the test results data.

Fig. 20 is an illustrative test profile.

Fig. 21 illustrates a display of test data including quality and correct action indications.

Fig. 22 illustrates the computer logic on pseudo code used in a typical test involving four tests.

Fig. 23 shows a test profile involving the use of two batting tees.

Fig. 24 shows a test profile of a catcher pop-up drill.

### DETAILED DESCRIPTION OF THE INVENTION

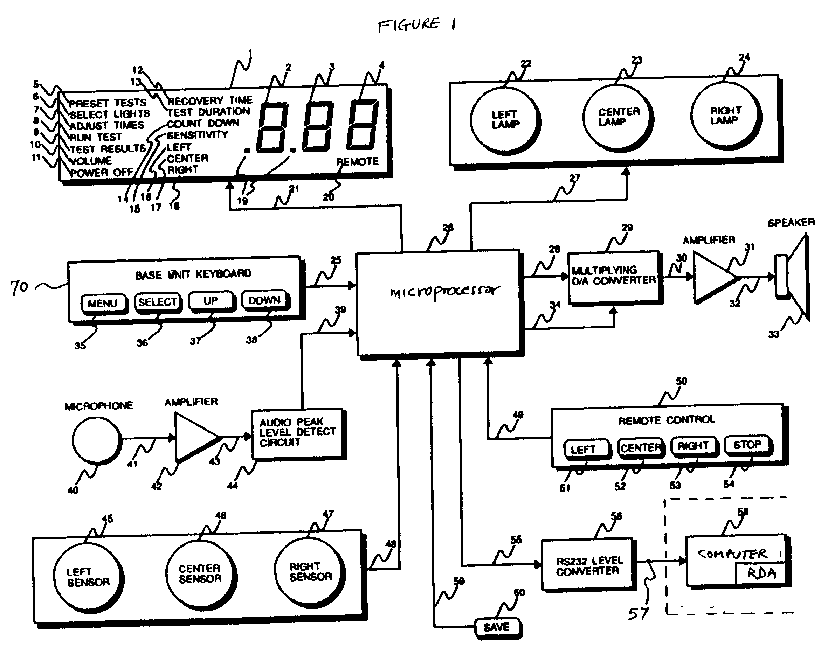

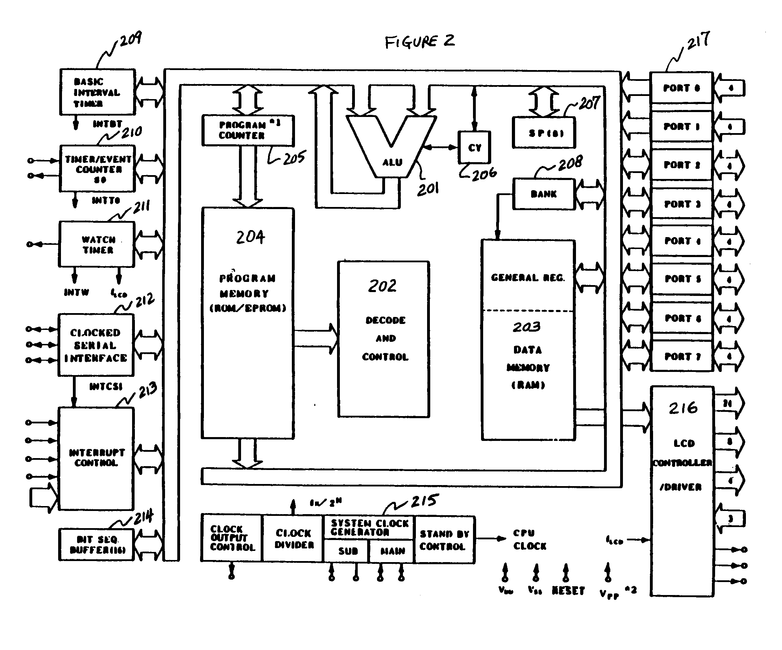

Fig. 1 is a block diagram of the Advance Reaction Trainer according to the present invention. A microprocessor 26 implements many of the functional aspects of the invention. A commercially available NEC 75308 microprocessor, or any equivalent microprocessor, may be used in a preferred embodiment. The major components of the NEC 75308 includes program and data storage memory, input and output ports including a serial interface for downloading memory data to an external device, a plurality of timers, and a liquid crystal display controller. The components of the microprocessor 26 and its interconnects to external components will be discussed in more detail below and in Fig. 2.

A liquid crystal display 1 is an output interface to the user. In a preferred embodiment the display 1 includes three numerical digits 2, 3 and 4, decimal points 19 and 19A and first and second columns of texts. The numerical digits and the decimal points represent a variety of parameters and data. The first column of texts is a menu of functions 5 to 11. These functions are user selectable and include functions: 5 for PRESET TESTS; 6 for SELECT LIGHTS; 7 for ADJUST TIMES; 8 for RUN TEST; 9 for TEST RESULTS; 10 for VOLUME; and 11 for POWER OFF. Each of these functions will be further discussed below.

The second column of texts indicates parameters 9 to 15 and direction 16 to 18. Parameters 12 to 15 are adjustable when used in conjunction with the ADJUST TIMES 7 function. The parameters are: RECOVERY TIME 12; TEST DURATION 13; COUNT DOWN 14; and SENSITIVITY 15. Direction indicators 16, 17, 18 are used to indicate the digits 2, 3, 4 being displayed are for a LEFT 16, CENTER 17 or RIGHT 18 action. A separate word 20 indicates when the unit is in the REMOTE mode.

Each segment pin of the display 1 is connected to a segment driver pin of the microprocessor 26 through a conductor 21. This arrangement allows the software to individually control each segment of the display 1 in order to provide the correct visual indication of the current operating mode.

Keyboard 70 includes four keys 35, 36, 37, 38, which may be momentary switches. The keys 35 to 38 are used to select functions, adjust parameters as well as review data. The keyboard 70 is connected to the microprocessor 26 through a conductor path 25. This arrangement allows the software to monitor the keys to determine the user's request.

A plurality of lamps 22, 23, 24 are placed substantially evenly across a horizontal plane. The lamps are controlled by the microprocessor 26 through conductor path 27 as needed to perform the various tests.

A microphone 40 converts audio signals into an electrical signal which is amplified by the amplifier 42 before it is fed to the audio peak level detection circuit 44. This type of circuit is commonly used in audio equipment to create a "BAR GRAPH" level meter. The output of the detection circuit 44 is connected to an input port of the microprocessor 26 via conductor data path 39. This arrangement allows the microprocessor 26 and its associated stored programs to monitor the audio level to determine whether an action has taken place. For example, the impact of a bat hitting a ball.

A speaker 33 provides audio feedback to the user. The speaker is driven through a conductor path 32 by an amplifier 31, which is in turn driven by a digital to analog (D/A) converter 29 through conductor 28. The reference voltage input of the D/A converter 29 is connected to a buzzer output port of the microprocessor 26 through a conductor 34. The digital input of the D/A 29 is connected to an output port of the microprocessor 26. This arrangement allows the user to control the volume of the beeper or buzzer.

According to an alternative embodiment of the present invention, sensors 45, 46, 47 provide alternative methods of detecting an action. When the microprocessor 26 determines that the sensors 45, 46, 47 are installed by way of a cable 48, the microprocessor 26 ignores the audio peak detector output 44 and looks for signals from the sensors 45, 46, 47 to determine the end of an event. This arrangement allows the optional use of various other sensors such as foot switches, optical interruption, etc.

The remote assistant coach device 50 provides an optional method of controlling the lamps 22, 23, 24. The switches 51, 52, 53, 54 are connected to an input port of the microprocessor 26 by a six conductor cable 49. When the microprocessor 26 determines that the REMOTE 50 is installed, it energizes lamps 22, 23, 24 in accordance with the activation of the START/STOP switch 54 or switches 51, 52, 53.

The STOP 54 button stops the timer within microprocessors 26. The remote assistant coach device 50 overrides the operation of the audio peak detector 44 and the optional sensors 45, 46, 47. The remote assistant coach device 50 operates as a "directional stopwatch". When the remote device 50 is connected to the ART, the microprocessor 26 will set the display 1 so that the REMOTE 20 text will be lit. The remote device 50 may also communicate with the ART by using alternate connection means such as a RF or an infrared link known to one skilled in the art.

The microprocessor 26 communicates with an external device, such as a personal computer, through serial port 72, RS232 level converter 56 and RS232 cable 57. Activation of a SAVE switch 59 causes transfer of stored data including response time data to an external computer device 58.

The software program for controlling most of the microprocessor operations of the Advanced Reaction Trainer according to the present invention resides in the read only memory(ROM) associated with the microprocessor 26. The random access memory(RAM) associated with the microprocessor 26 stores variable data including response-time data.

Fig. 2 is a schematic block diagram of the components of microprocessor 26 and its interconnects according to one embodiment of the present invention. The Arithmetic Logic Unit (ALU) 201 provides capability for logical and arithmetic calculations. Data memory (RAM) 203 provides random access storage of data including program variables or user's response-time. Program memory 204 is a read only memory (ROM) for storing software program routines including routines to direct the operation of the hardware components of the ART, for sequencing through the different functions of the ART and for adjusting the different parameters of the ART. A programmable read only memory (PROM) instead of a ROM may also be used.

Three separate timers 209, 210 and 211 are provided. Timer 210 and Interrupt Control 213 can provide an interrupt every one thousandth of a second. Timer 211 is connected to the D/A converter 29 to activate beep signals. Clocked serial interface 212 connects to the RS232 level converter 56 for outputting data stored in RAM memory 203 to computer 58. The system clock generator 215 provides a system clock for system timing. Preferably, a 3.58 megahertz crystal connects to the system clock generator 215.

LCD controller and driver 216 converts information output from RAM memory 203 into drive signals for energizing the corresponding segments of the LCD display 1. Input/Output port 217 serves to interface other components with the microprocessor 26 including: save button 60 at port 0; keyboard 70 at port 1; the remote assistant coach device 50 at port 2; audio peak detector 44 at port 4; light output controls at port 5; and input/output ports 6 and 7 are reserved for optional sensory equipment such as touch pads.

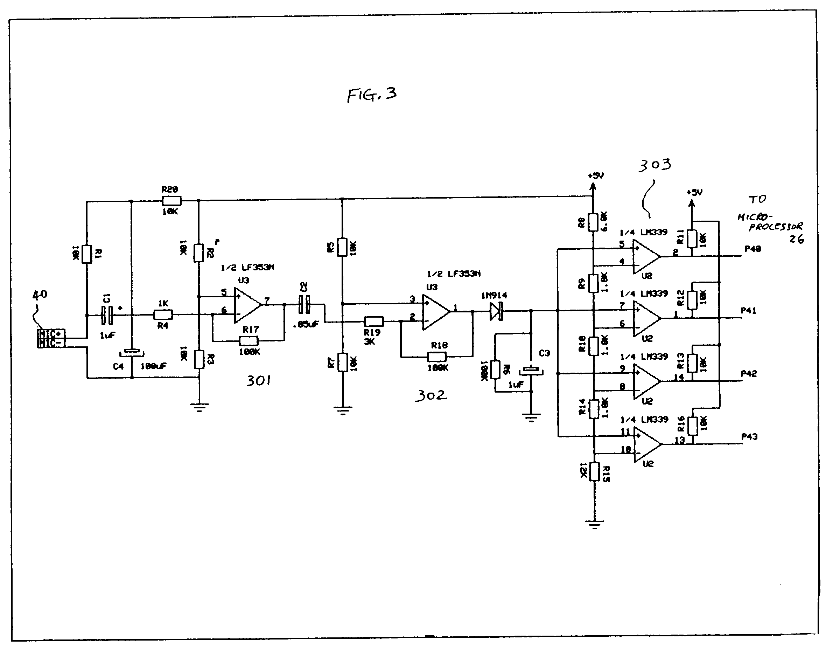

Fig. 3 is a schematic diagram of the audio peak detector 44. When used in conjunction with microphone 40, the detector activates output signals to indicate an acoustic level picked up by the microphone 40 which exceeds a preset threshold value. As shown in Fig. 3, two stages of amplifiers 301 and 302 amplify the signal detected by the microphone 40. A set of four comparators 303 receives the amplified signal. Each comparator is set at a different threshold, and if the amplified signal exceeds any threshold setting, a high level signal appears at the output of the corresponding comparator.

Each of the outputs of the comparators may be connected to the I/O port 217 of microprocessor 26. When the appropriate software routine is activated, this I/O port 217, for example, port 4 will be sampled every one thousandth of a second, and if a high signal is detected at port 4, the microprocessor 26 will stop the response-time timer.

The software program which operates the components of the ART resides in the memory 203 and/or 204. The software program also includes an associated software routine for each of the functions 5 through 11. The user selects the function to be executed by pressing the MENU button 35 on keyboard 70. Routines for functions RUN TEST 8 or POWER OFF 11 are not activated unless the SELECT button 36 is depressed.

The routine associated with function ADJUST TIMES 7 allows the user to adjust the four parameters RECOVERY TIME 12, TEST DURATION 13, COUNT DOWN 14 and SENSITIVITY 15. Recovery Time 12 is the time between the response and the next stimuli or activation of the next light. Test Duration 13 is the user adjustable amount of time allocated for the entire exercise. Count Down 14 activates a set sequence, for example, a display of 3-2-1 within a user selectable time period prior to the activation of the stimulus. Sensitivity 15 allows the user to select one of four threshold levels at the acoustic detector 44. The user selects or steps through a sublevel routine for each parameter by pressing the SELECT button 36. The currently selected parameter value is displayed by the digits 2, 3 and 4 of the display 1. These values can be adjusted by the user with the UP 37 and DOWN 38 buttons. Other displayed values, such as values for functions VOLUME 10 or TEST RESULTS 9, may be similarly adjusted by the UP 37 and DOWN 38 buttons.

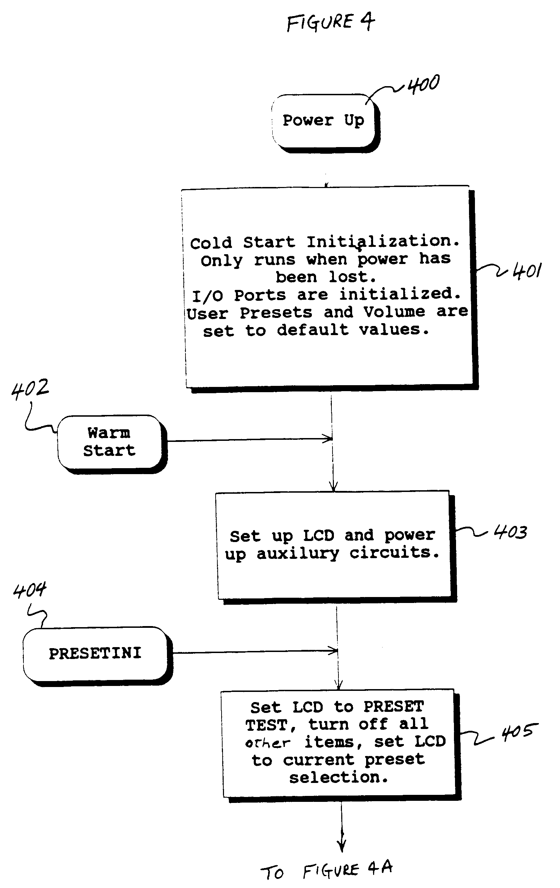

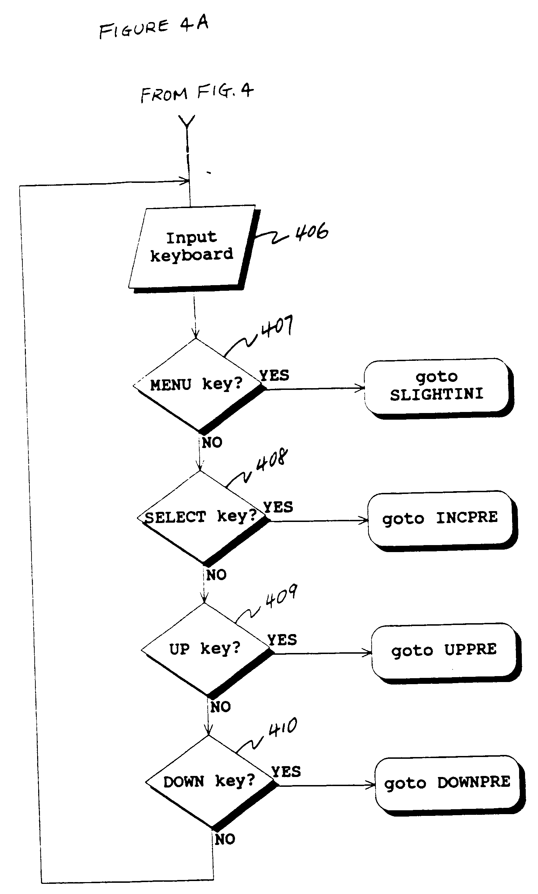

Referring to Fig. 4, upon turn on of the ART, the Power Up routine 400 is executed, the microprocessor 26 causes the software to be started at its reset vector. This vector points to the initialization software which sets up all of the necessary default parameters in their appropriate RAM locations such as initializing the preset memory to known values for all of the parameters of all nine presets. In a preferred embodiment, each preset contains five parameters: number of lights, recovery time, countdown time, test duration and sensitivity. This routine also initializes the Volume setting, light pattern pointer and test results pointer (step 401). The software will then jump to the Warm Start routine 402.

The Warm Start routine initializes the display 1 and set preset equal to "1". The temporary variables are then set to values previously stored with preset "1". The display 1 is turned on so that the unit is ready to accept user requests in routine step 403.

Execution of the preset test routine 404 turns on the PRESET TEST 5 function of the display 1 and turns off all other functions (6 through 18). The currently selected preset will be displayed in the three digits 2, 3, 4 of display 1. The software will then enter into a loop which waits for one of three user requests of keys 35, 37 and 38 of keyboard 70 by sampling the input port associated with conductor 25 (step 406). If the MENU switch 35 is pressed (step 407), this routine jumps to the Select Lights Routine.

If either the UP 37 or the DOWN 38 key is pressed at steps 409 and 410, the software changes the PRESET number associated with digit 3. Each time the UP 37 key is pressed, it will cycle up the value displayed with digit 3 from "1" to "9" and then back to "1" and so on. Each time the DOWN 38 key is pressed, the software cycles down the number displayed by digit 3 from "9" to "1" and back to "9" and so on. This allows the user to select one of nine presets which contain values for parameters such as number of lights, recovery time, test duration and count down value. When there is a change in the preset value, the new value is stored in the RAM memory 203 in locations allocated for these parameters. If the switches 35, 37 or 38 are not pressed, the software will jump back to the beginning of the loop.

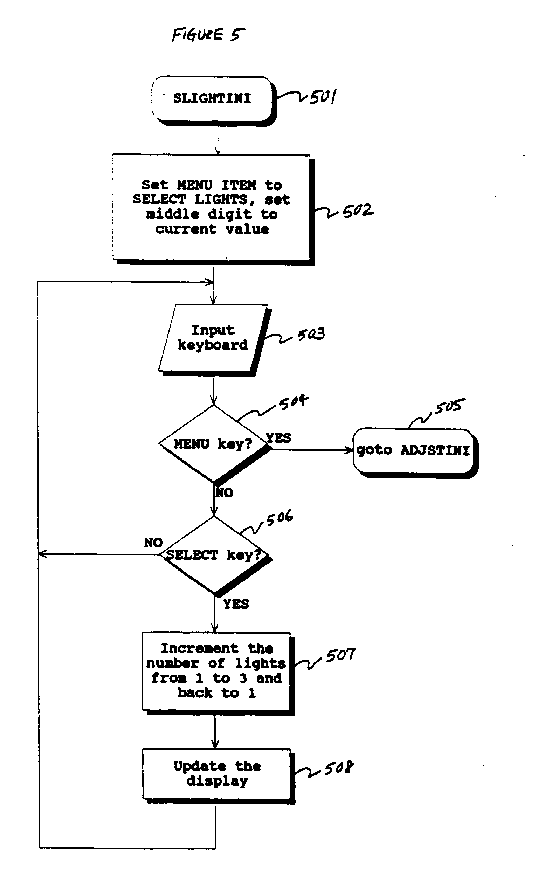

Referring to Fig. 5, execution of the Select Lights routine 501 turns on the function SELECT LIGHTS 6 of the display 1. The current selected number of lights will be displayed by digit 3 of display 1. The program then waits in a loop for a light selection signal from the keyboard 70 (step 503). If the MENU key 35 is pressed, the routine jumps to the Adjust Times routine 505.

If the SELECT key 36 is pressed, it cycles up the value displayed at digit 3 from "1" to "3" and then back to "1" and soon as in step 507. In addition, the location of RAM memory 207 that indicates the current number of lights to use in the test is changed. If neither switches 35 or 36 is pressed, the program jumps back to the beginning of the loop.

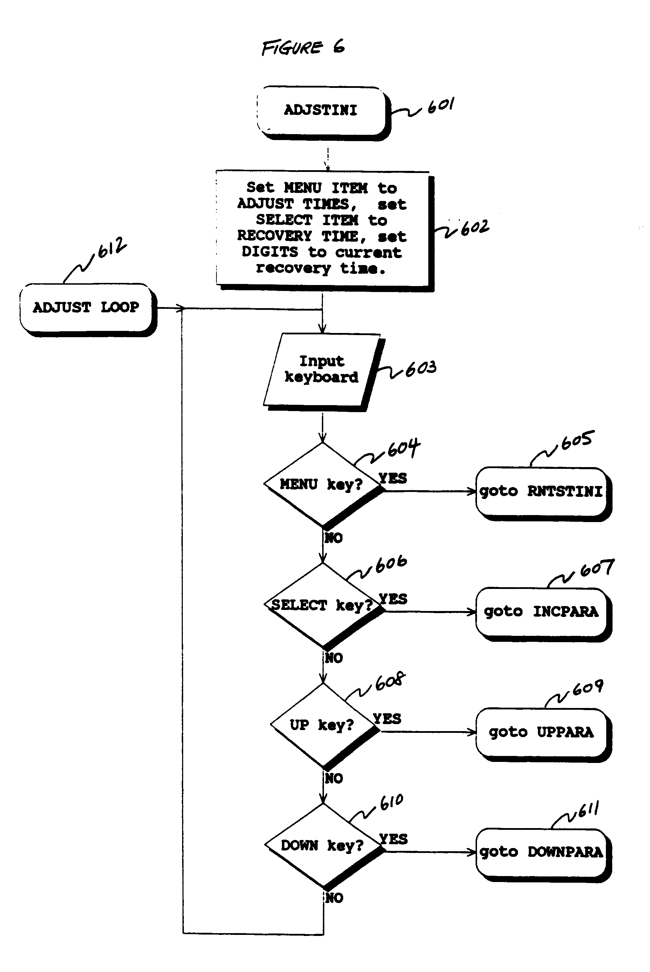

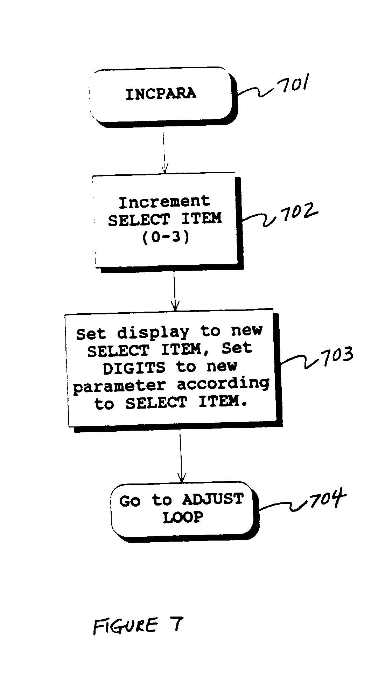

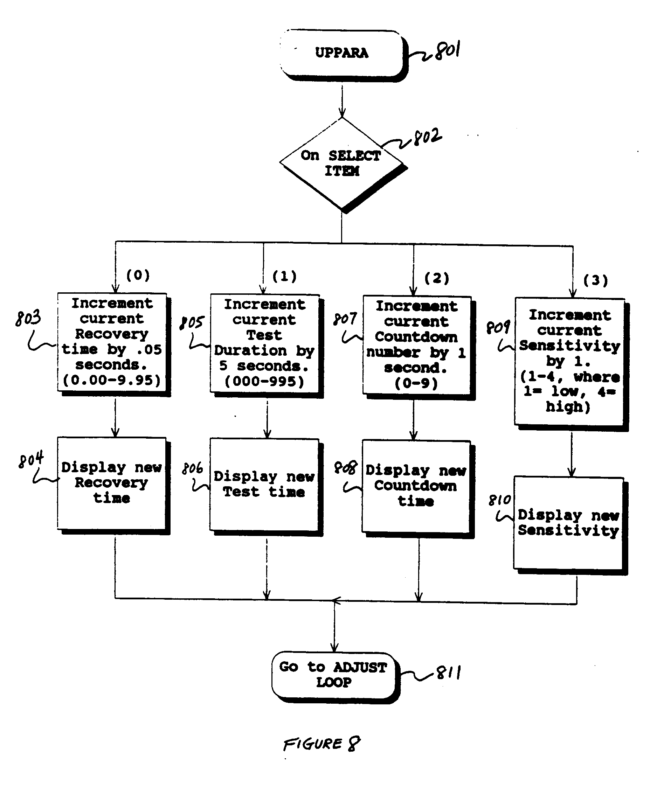

Referring to Fig. 6, the Adjust Times routine 601 turns on the ADJUST TIMES 7 function and the RECOVERY TIME 12 parameter of the display 1. In step 602, the current recovery time value will be displayed by the three digits 2,3 and 4 of display 1. Then the routine waits in a loop and samples the keyboard connection 25.

If the MENU key 35 is pressed in step 604, the current parameter values are transferred to the location of the RAM allocated for the corresponding parameter. With the above approach, the user may modify the parameter values of any preset and have them automatically recorded for recall later. After completing the above, the software jumps to the Run Test routine in step 605.

In step 606, if the SELECT key 36 is pressed, the software turns on the next parameter name and cause the three digits 2, 3 and 4 to display the value of that new parameter. For example, if the RECOVERY TIME segment 12 was on, the software turns it off and turn on the TEST DURATION segment 13, and causes the three digits 2, 3 and 4 to display the current test duration value. The order of parameters is RECOVERY TIME 12, TEST DURATION 13, COUNT DOWN 14 and SENSITIVITY 15.

If either the UP 37 or the DOWN 38 keys is pressed in steps 608 or 610, the routine increments or decrements the current parameter value in the RAM memory 207, as well as update the three digits 2, 3 and 4 on display 1 to indicate the value in the RAM. For example, if the RECOVERY TIME segment 12 is on, then the current value for recovery time is displayed by the three digits 2, 3 and 4. If the UP key 37 is pressed, the routine will increment the current recovery time value by .05 seconds and digits 2, 3 and 4 will be changed to indicate the new value of the current recovery time. If none of the keys 35 through 37 are pressed, the software will jump back to the begining of the loop.

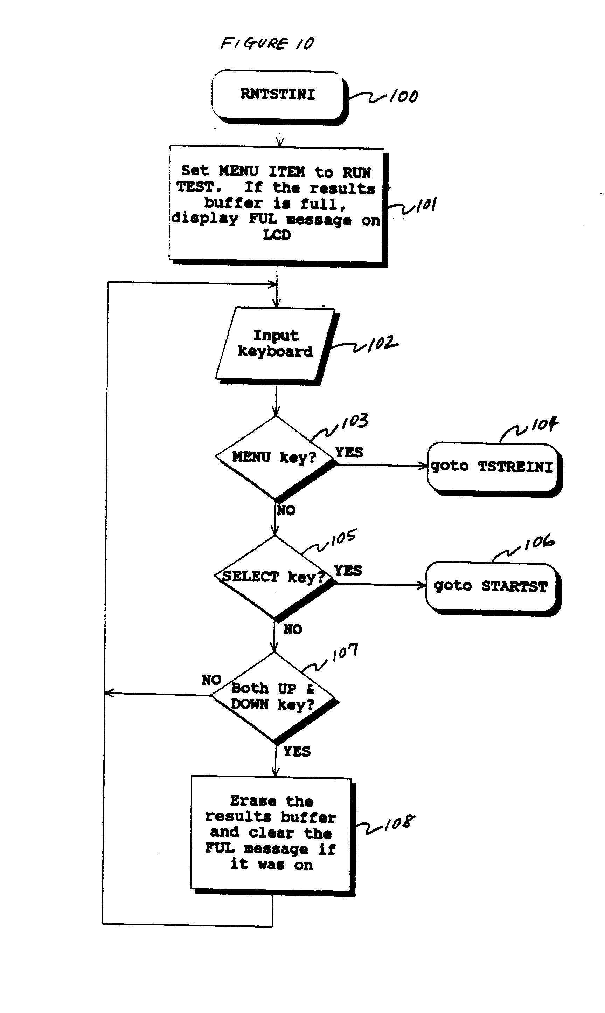

Referring to Figure 10, which shows the flow diagram for the Run Test routine, at step 100, RUN TEST 8 function of the display 1 is turned on. All other functions are turned off unless the test results locations of the RAM memory 207 is full, in which case, the digits 2, 3 and 4 indicate the full condition by displaying "FUL". The user then erases the old test results before they run another test.

In step 102, the routine samples input 25 from the keyboard. If the MENU key 35 is pressed (step 103), the routine jumps to the Test Results routine. If the SELECT key 36 is pressed (step 105), the routine enters a test sequence corresponding to the current parameters. A new subject number will be assigned in the results area of the RAM memory 207 so that the subsequent test results will be separated by this subject number. A duration time timer which limits the duration of an exercise may be set and started. If both the UP 37 and the DOWN 38 keys are pressed substantially simultaneously (step 107), the software will erase the previous test results and clear the FUL message if it was on (step 108). If none of the keys 35 through 37 is pressed, the software jumps back to the beginning of the routine.

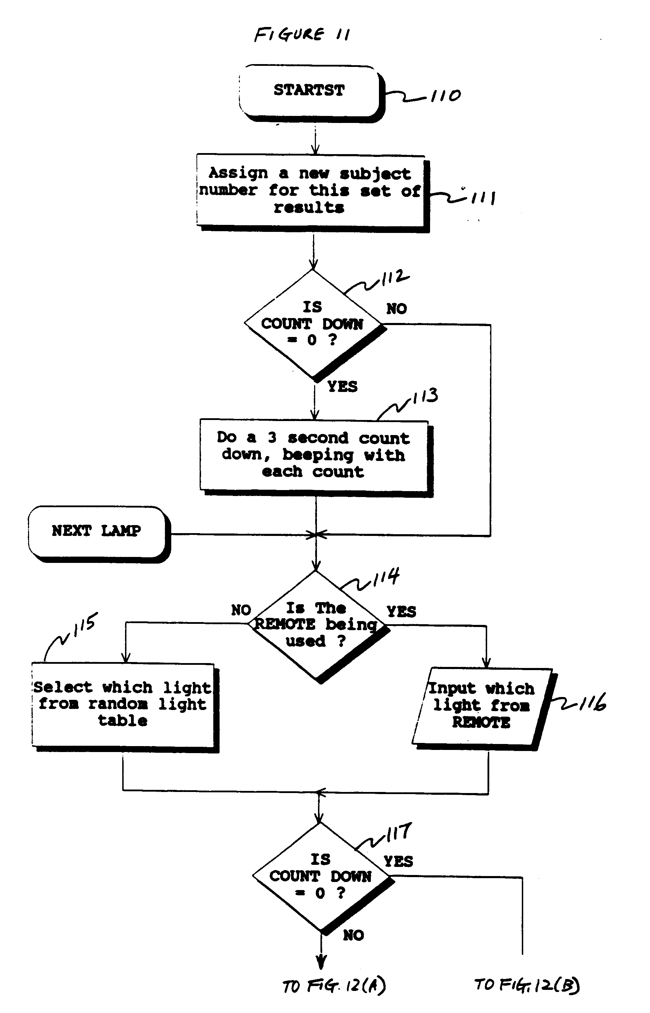

Figure 11 illustrates a typical flow of a Run Test routine. In step 111, a new number is assigned to the user. If count down is nonzero (step 112), the ART will perform a count down sequence. The count down feature of the present invention may be adjustable between one to five seconds. If the current count down value is not zero, a count down will occur to prepare the user for the illumination of a light. For example, if the current count down value is 5, then the unit will beep as well as count down digit 3 of display 1 from 3 to 1 over a duration of five seconds before lighting a selected lamp. No count down signal will precede the initiation of a light if the countdown value is zero. In step 113, the countdown is set to 3. Accordingly, the beeper and the display will beep and count at one second intervals.

The Next Lamp Loop subroutine randomly lights one of the lamps. If the assistant coach remote control 50 is connected (step 114), lamp selection is controlled by the remote buttons 51, 52 and 53. When any lamp is lit, two software timers are started. One timer is started to determine the time that the lamp remains lit (step 119). A second timer is started to measure the user's response time. To capture the user's response time, the audio peek detector 44, the optional sensors 45, 46 and 47, or the assistant coach remote control 50 is monitored by the microprocessor 26 dependent on the configuration of the system (steps 122 to 128). The receipt of a response from any of the above components causes the 1/1000 second interrupt to stop counting response time at the microprocessor 26, the response-time timer is then read and stored in a designated location in RAM memory 207. Thereafter, if the lamp-on timer has expired, the lamp is turned off. If the user does not respond within 9.95 seconds after the turn on of the lamp, the response-time timer stops and indicates 9.95 seconds as if the user responded at 9.95 seconds. Both the lighten timer and the response-time timers may be a hardware timer such as a timer 210 or a software timer monitored by the software.

The user's response time is stored in the next available location of RAM memory 207 designated for the test result values (step 129). The response time is also displayed on the three digits 2, 3 and 4 of display 1. The duration time timer is checked to determine if the time has expired and the exercise should then be ended. If not, the software waits until the expiration of the recovery time before jumping to the next lamp loop. If the assistant coach remote control 50 is connected, the software jumps back to the next lamp loop without waiting for the recovery time.

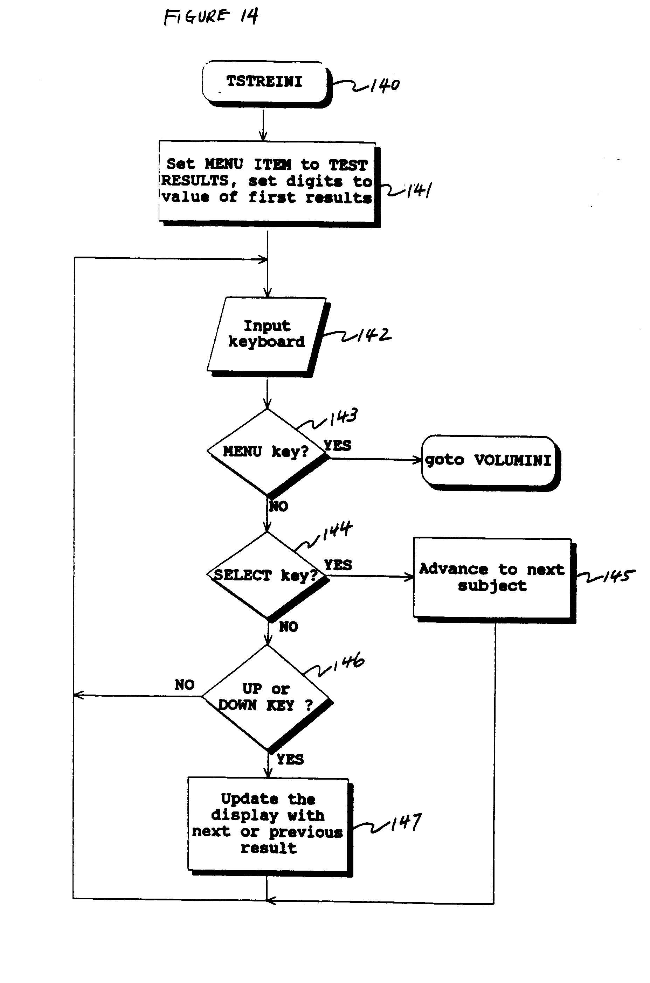

Figure 14 illustrates the flow diagram of the Test Results routine. At step 140, the TEST RESULTS 9 function of the display 1 is turned on. The first test result value is displayed on the digits 2, 3 and 4. The appropriate segment LEFT 16, CENTER 17 OR RIGHT 18 of the display 1 is turned on to indicate which lamp was lit at the time of the test. The routine then enters a waiting loop and monitors requests from the keyboard 25 (step 142).

If the MENU key 35 is pressed (step 143), the program jumps to the VOLUME ROUTINE. If the SELECT key 36 is pressed (step 144), the program advances the test result pointer to the next user. A user is indicated by digits 2, 3 and 4 displaying - 1-, -2-, etc. (step 145). If either the UP 37 or the DOWN 38 key is pressed (step 146), the routine increments or decrements the pointer to the test result values in the RAM memory 207 and updates the three digits 1, 2, 3 on the display 1 to indicate the test results (step 147). The user may view the entire list of results by stepping the UP 37 or DOWN 38 key. If none of the keys 35 through 38 are pressed, the program returns to the beginning of this loop. The test results may also be downloaded to an external device. This process will be further discussed below.

The Volume routine 150 turns on the VOLUME 10 function of the display 1. The current volume parameter value is displayed by digit 3 of the display 1 (step 151). The routine then waits in a loop for an input from the keyboard 70. If the MENU key 35 is pressed (step 153), the program jumps to the Power Off routine.

If either the UP 37 or the DOWN 38 keys is pressed (step 154), the routine increments or decrements the current volume parameter value from 0 through 9 or 9 to 0 (step 155). Each time the user changes the volume parameter, the microprocessor 26 updates the D/A converter 29 accordingly (step 156). If none of the keys 35, 37 or 38 is pressed, the routine will jump back to the beginning of the loop.

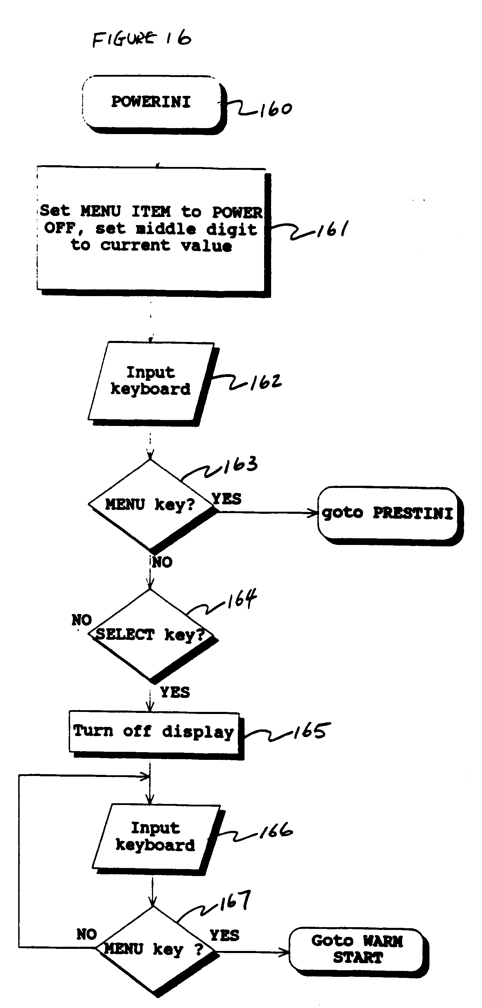

The Power Off Routine is illustrated in Figure 16. At step 160, the POWER OFF 11 function of the display is turned on. The keyboard 70 is sampled. If the MENU key 35 is pressed, the program jumps to the Preset Test routine (step 163). If the SELECT key 36 is pressed (step 164), the display shuts off and the program waits for further commands from the keyboard 70. If the MENU key 35 is pressed, the program goes to the Warm Start routine. If neither keys 35 or 36 is pressed, the routine jumps back to the beginning of this loop.

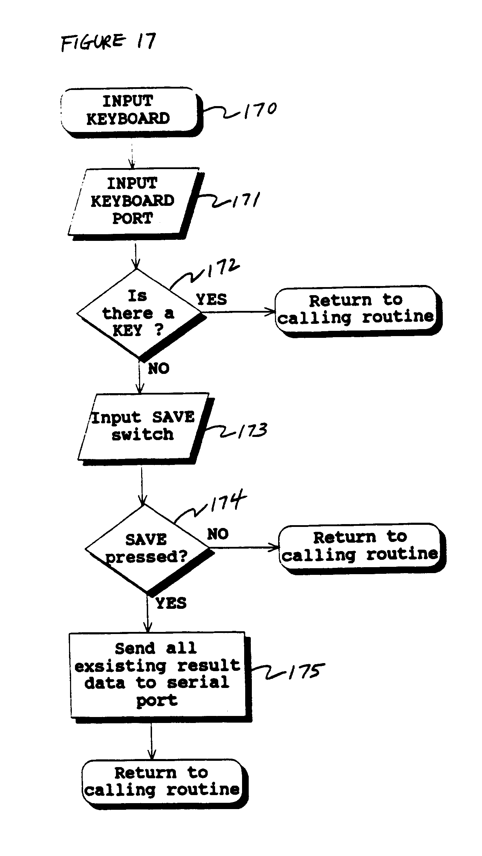

In the Computer Interface Loop, illustrated in Figure 17, whenever the input ports for the keys 35 through 38 of the keyboard 70 are sampled, another routine simultaneously samples the SAVE key 60. If the SAVE key 60 is pressed prior to any keys 35 through 38 (step 174), the microprocessor 26 locks out the keyboard 70 and downloads or outputs the test result data stored in the RAM memory 207 to an external device through the RS232 level converter 56 and cable 57 (step 175). The external computer 58 is connected through conductor 57 and executes the required RS232 protocol, the RAM data will be captured by the computer 58 for further processing and analysis. After completion of the downloading of the RAM data, the routine releases the keyboard lockout and returns to its previous function.

Data processing and analysis, including computation of the data into reaction factors, is another aspect of the present invention. A Reaction Data Analysis module ("RDA") of the external computer 58 analyses and quantifies the data downloaded from the RAM memory 207. The computer 58 performs other tasks including retrieval of test results, entry and storage of empirical data correlating to game situations, and store or update the user's personal data.

The logical components of the RDA module include: an interface component which manages the interaction and transfer of response-time data from the ART to the RDA; a conversion component which changes the internal response-time data format of the ART into a format which can be used for general computer processing and analytic analysis; entry and comparison of stored data including empirical data correlating to game situations with profiles, accuracy (correct target) and quality of reaction information; a filter component which eliminates potentially erroneous reaction data; a computational component which computes reaction factors for each light for each test and computation of an overall reaction factor; a spreadsheet component capable of displaying printing or further analyzing the data with a table of spreadsheet data; and a storage and update component which stores the original and computed reaction or response-time data in a historical database which can be accessed for future personal progress comparison or for advanced trend analysis.

The interface component transfers reaction data between the RAM 203 of the ART and the external computer 58 via the RS232 serial cable 57. The cable includes a special connector on one end which plugs into the ART and a standard 9 pin serial connection on the other end for plugging into a standard 9 pin serial port on the external computer 58.

The RDA interface component sends special characters to initiate the sending of information by the ART. The special characters include any bit pattern which causes the Clear-to-Send to change levels. This signals the ART to start the transfer. The reaction data in RAM 203 is sent as a sequence of 7 bit ASCII bytes with one stop bit and no parity. After all information bytes have been transferred, a checksum and an ASCII end-of-file character is transferred. The RDA interface component computes the checksum on the transferred data and compares it to the checksum transmitted by the ART. If the checksums do not match, the RDA initiates the transfer again. This is repeated for N times, where N is a system option in the RDA, which is set to 3 in the preferred embodiment. If, after N attempts at transferring the data, the checksums are still different, an error message is displayed on the screen of the external computer 58.

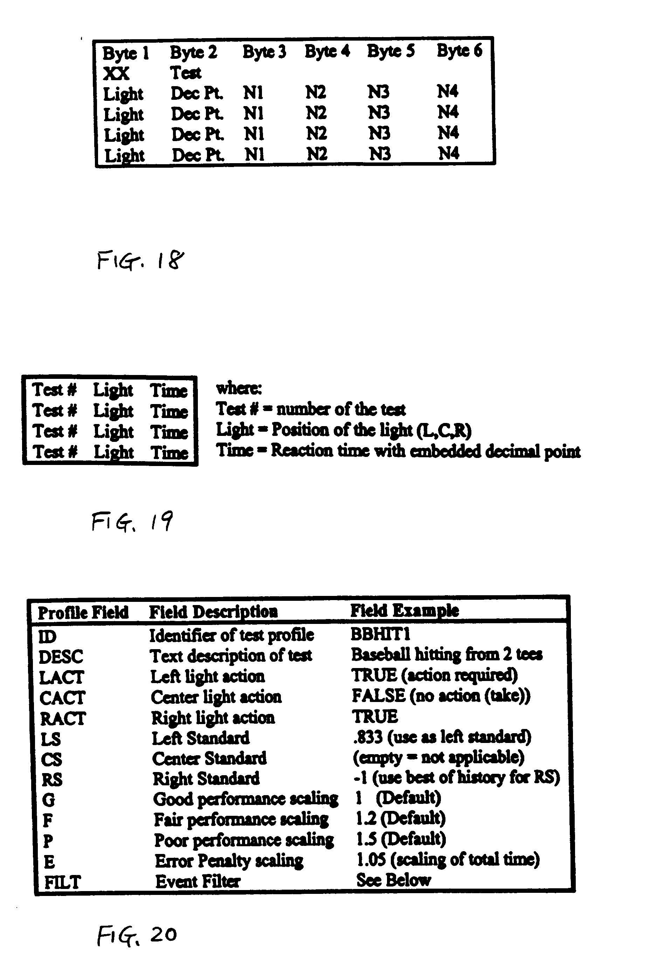

Upon receipt of the reaction data from the ART, the reaction data conversion component of the RDA within external computer 58 converts the reaction data to a format suitable for general purpose data processing. The downloaded reaction data represents the results of one or more tests. A header record indicates the beginning of a test. Each set includes a sequence of timed reaction. Each response-time or reaction data includes information about which light on the ART was illuminated and the reaction time. The recorded response time is represented by a decimal point indicator and four digits of resolution. Figure 18 shows a test of four reaction times consisting of a header record and four time records, each of 6 bytes. Where: XX = special New Test code; Test = Test Number (0-9); Light = Light(L,C,R); Dec. Pt. = Decimal Point; and N1 .. N4 = 4 digits.

The test data is converted to records of three values each, representing the test number, light position and response time in real number with an embedded decimal point. This data is written to a file as ASCII characters in a comma separated value format with each record terminated by ASII Carriage Return, Line Feed end of line characters. This format is suitable for reading data into general purpose analysis software, such as spreadsheets. Figure 19 shows the converted data representation of the previous figures data.

The computer 58 allows the user to enter additional data including test identifier to retrieve a profile and history of the test; empirical data which correlates to game situations; and information about a test and observations about events within a test.

Each test may include a profile attached which describes the test for the reaction factor computation. This profile is associated with a unique identifier. For example, BBHITI may be the identifier for a baseball hitting test 1. The user can look through a directory of identifiers and descriptions of existing profiles as well as create new profiles. If an existing identifier is entered, the profile is retrieved and made available for modification. If a new identifier is entered, a blank profile form is provided for entry of data. Fig. 20 illustrates an example of a test profile identified by BBHITI.

The event filtering is used by the filter and analysis component of the RDA to improve the quality of the reaction factor computation. The filtering options are "0" for no filtering; "1" for removal of the best and worst events of the test; "2" for removal of the first event only and so on. The filter may also be identified by NAME, which represents the type of filtering, such as a statistical filter.

To improve the reaction factor calculation for the recorded response-time tests, each event of a test may include user input to indicate the quality of the reaction, such as G = good, F = fair and P = poor. Similarly, there may also be a Correct Action field where "Y" indicates a correct reaction and "N" indicates an incorrect one. If CORR and QUALITY factors are not entered for an event, the default (CORR=Y and QUALITY=G) is applied.

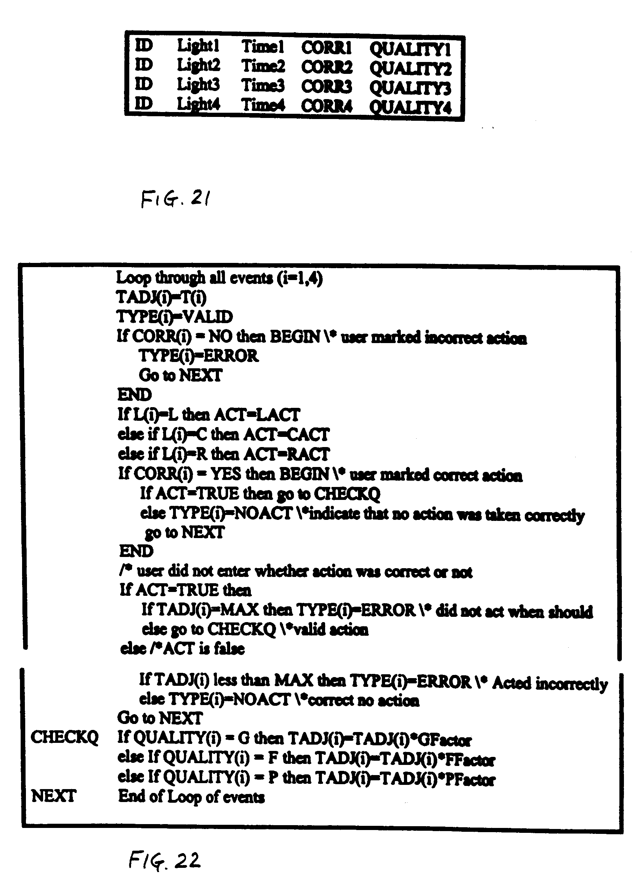

Fig. 21 illustrates the test result with Corr and Quality indicated.

Fig. 22 shows the computer logic in pseudo code used in a typical test involving four events. For each event, the time, T(i), is adjusted to a new value, TADJ(i), TYPE(i) is marked as either VALID, ERROR, or a correct NOACT (no actions). MAX is the maximum time allowed for the event. L(i) and T(i) represent the light position and time for event (i), CORR (i) and QUALITY (i) represent the correct action and quality for event (i).

The next step is to filter the reaction data as specified in the profile. If FILT = 0 then go to EXIT because no filtering is specified; if FILT is a name, call the named routine to filter the data; if FILT = 1 then loop through the data and remove the highest and lowest number; and if FILT = 2 then remove the first event data.

To quantify the reaction data into a "factor", the following computations are made: (1) An average Reaction Time, RT, is defined as the sum of a set of reaction times divided by the number of times in the set; (2) A standard Time, ST, is defined as the standard reaction time. The ST can be derived from a number of sources depending on the requirements of the test, including read from the test profile as a known and fixed standard; computed from test data as the best or lowest value in history; or computed from test data as the next in historical trend of test data; and (3) The Reaction Factor, RF, is computed as the ratio of ST divided by RT.

Thus, reaction data, related quality information, reaction times, reaction standards and reaction factor are all stored for each test in a relational database to provide a history of reaction information. This data can be retrieved for display or computation by type of test or by user.

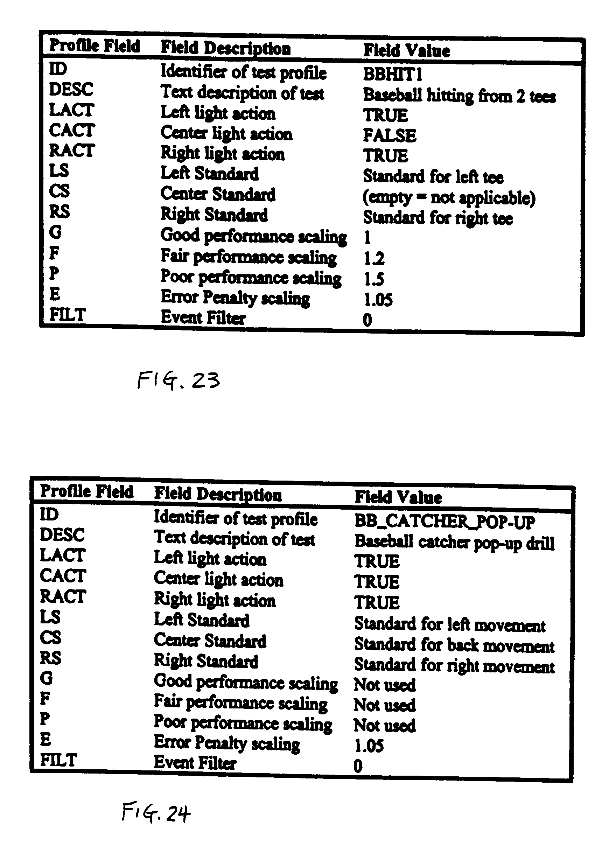

The stored data is applied to a game situation baseball batting drill with the ART. For example, the test involves the use of two batting tees. The ART is programmed to display all three lights. The left light indicates that a ball should be hit on the left tee, the center light indicates a take, and the right light indicates that a ball should be hit on the right tee. The test may include the "count down" sequence to simulate pitcher windup; the use of the acoustic impact detector; or the "assistant coach" device to record response-time data. The profile for the test is shown in Fig. 23.

The events can be further qualified with annotations such as: Y = correct tee or take; N = incorrect tee or swing when should be take for Corr Action; G = Solid Hit; F = Pop-up or topped ball; and P = Missed ball for the Quality field.

Another example, illustrated in Fig. 24 is a catcher pop-up drill which involves timing the user's reactions when moving left, back or right when a light triggers the action. The ART is programmed to display all three lights. The catcher starts from a crouch position. The left light indicates that the catcher should move to a position to his left, the center light indicates a movement to a position behind the catcher, and the right light indicates that he should move to a position to the right. The "assistant coach" device is used in this drill to record response-time.

Although several embodiments of the invention have been illustrated in the accompanying Drawings and described in the foregoing Detailed Description, it will be understood that the invention is not limited to the embodiments disclosed, but is capable of numerous rearrangements, modifications, substitution of parts and elements without departing from the spirit of the invention.

## Claims

1. An apparatus for reaction training, having optical functions and parameters, comprising action indicator means for signalling a reactionary movement patern;
sensor means for detecting said reactionary movement; a timer for measuring the reaction time from the initiation of said action indicator means to said detection of said reactionary movement;
function selection means for selecting said optional functions;
adjust means for adjusting said optional parameters;
processor means for operation control including selective activation of said action indicator means;
a stored program memory for storing programs including user selectable function programs for execution by said microprocessor;
and a data memory for information storage including response time data.

2. An apparatus according to claim 1 including display means for displaying a selected function or the response time.

3. An apparatus according to claim 1 or 2 wherein said selectable functions include a count down function for signalling a preset count down sequence prior to the activation of said action indicator means for signalling movement patterns.

4. An apparatus according to any preceding claim including means for generating an acoustic impact for detection by said sensor.

5. An apparatus according to claim 4 wherein said sensor means includes an acoustic detector for detecting the sound produced by the impact.

6. An apparatus according to any preceding claim wherein said function selection means includes a remote device for selecting operation of said action indicator means and for stopping said response time timer.

7. An apparatus according to any preceding claim including:
means for downloading information stored in said data memory including response time data to an external processing device; and
means for quantifying said response time data into reaction factors.

8. An apparatus according to claim 7, wherein said external processing device includes a processor for communicating with said means for downloading and for processing said downloaded data; and
storage means for storing said downloaded and processed data.

9. An apparatus according to claim 8, wherein said processor generates an historical data base including said downloaded and processed data.

10. An apparatus according to calim 8 or 9, wherein said storage means includes empirical data for comparison with said downloaded data.

11. An apparatus according to any of claims 7 to 10 wherein said external processing device includes a display for displaying a spreadsheet of said processed data.

12. A method for reaction training comprising the steps of:
selectively energising a plurality of action indicators, each of said indicators corresponding to a different rection movement, comprising the steps of:
counting down for a selected time period prior to the energisation of said selected action indicator;
activating a timer when said selected action indicator is energised;
detecting said reaction movement in response to said energised action indicator and deactivating said timer upon said detection; and
recording the time between said energisation of said selected action indicated and said detection of said responsive reaction movement.

13. A method according to claim 12, wherein said detection of said reactionary movement includes detecting the sound produced by the impact from said reaction movement.

14. A method according to claim 12 or 13, further including downloading said recorded time to an external processing device.

15. A method according to claim 12, 13 or 14, further including the step of correlating said recorded time with stored empirical data in said external processing device.

16. A method according to any one of claims 12 to 15 including the step of generating a historial database from said recorded time.
